# EUROPÄISCHE PATENTSCHRIFT

(11) **EP 4 389 731 B1**
(45) Veröffentlichungstag und Bekanntmachung des Hinweises auf die Patenterteilung: **12.02.2025**
(21) Anmeldenummer: 22214450.3
(22) Anmeldetag: 19.12.2022
(51) Int. Cl.: C07C 45/50, C07C 47/02

(54) **VERFAHREN ZUR AKTIVITÄTSERHALTUNG BEI DER ABSTELLUNG DER HYDROFORMYLIERUNG**
METHOD FOR MAINTAINING ACTIVITY DURING HYDROFORMYLATION SHUTOFF
PROCEDE DE CONSERVATION DE L'ACTIVITE LORS DE LA SUPPRESSION DE L'HYDROFORMYLATION

(43) Veröffentlichungstag der Anmeldung: 26.06.2024
(73) Patentinhaber: Evonik Oxeno GmbH & Co. KG, 45772 Marl (DE)
(72) Erfinder: FRANKE, Robert, 45772 Marl (DE); ARSENJUK, Linda, 44369 Dortmund (DE); OTT, Florian, 45657 Recklinghausen (DE); SCHÜLLER, Jessika, 56767 Gunderath (DE); KREIS, Peter, 44227 Dortmund (DE); STENGER, Frank, 63755 Alzenau (DE); KRISTEN, Marc Oliver, 45721 Haltern am See (DE)
(74) Vertreter: Evonik Patent Association

(56) Entgegenhaltungen:
- EP-A1- 1 193 239
- EP-A1- 3 632 886
- CN-A- 1 736 602

## Beschreibung

Das Projekt, das zu dieser Patentanmeldung führte, wurde im Rahmen der Fördervereinbarung Nr. 869896 aus dem Forschungs- und Innovationsprogramm Horizon 2020 der Europäischen Union gefördert.

Die vorliegende Erfindung betrifft ein Verfahren zur Hydroformylierung von kurzkettigen Olefinen, insbesondere C2- bis C5-Olefinen in mindestens einem Reaktor, bei dem das Katalysatorsystem heterogenisiert auf einem Support aus einem porösen keramischen Material vorliegt und wobei bei der Abstellung des Verfahrens die Verfahrensschritte a) bis c) nach Anspruch 1 durchgeführt werden.

Die Hydroformylierung ist mit einer jährlichen globalen Produktionskapazität von mehreren Millionen Tonnen eine der bedeutendsten Reaktionen in der großtechnischen Chemie. Dabei werden Alkene (Olefine) mit einer Mischung aus Kohlenmonoxid und Wasserstoff (auch: Synthesegas oder Syngas) unter Verwendung eines Katalysators zu Aldehyden umgewandelt, die wichtige und wertvolle Zwischenprodukte bei der Herstellung von chemischen Massenprodukten wie Alkoholen, Estern oder Weichmachern sind.

Die Hydroformylierung wird im großtechnischen Maßstab ausschließlich homogen katalysiert durchgeführt. Die löslichen Übergangsmetallkatalysatorsysteme basieren üblicherweise auf Cobalt oder Rhodium, welches oft mit Phosphor-haltigen Liganden, bspw. Phosphinen oder Phosphiten, für die Hydroformylierung von eher kurzkettigen Olefinen eingesetzt wird.

Die Probleme bei den bekannten Verfahren sind vielfältig, wobei diese Probleme insbesondere damit verknüpft sind, dass sowohl Rhodium als auch Cobalt und deren Verbindungen vergleichsweise teuer sind. Es wird ein hoher energetischer und verfahrenstechnischer Aufwand betrieben, um Katalysatorverluste während des Hydroformylierungsprozesses weitestgehend zu vermeiden, beispielsweise durch teils sehr aufwändige Katalysatorrecyclingschritte. Zudem werden Produktaufreinigungsschritte aufwendiger, um sicherzustellen, dass möglichst keine Katalysatorrückstände im Produkt verbleiben.

Weitere Probleme bei den bekannten homogen katalysierten Verfahren sind die Stabilität der Liganden, die die Bedingungen der Hydroformylierung, wie Temperatur, Druck, pH-Wert, usw., überstehen müssen, und der Verbrauch von dem verwendeten Lösemittel während des Prozesses, der durch Nachdosieren ausgeglichen werden muss.

Um die vorgenannten Probleme bei der homogen katalysierten Hydroformylierung zu umgehen, sind Hydroformylierungsverfahren entwickelt worden, bei denen das Katalysatorsystem heterogenisiert wird, insbesondere durch Immobilisierung auf einem Trägermaterial. Die Begriffe Heterogenisierung und Immobilisierung sind demnach so zu verstehen, dass der Katalysator durch Ausbildung eines dünnen Flüssigkeitsfilms mithilfe einer ionischen Flüssigkeit auf der Oberfläche und/oder in den Poren eines festen Trägermaterials immobilisiert wird und keine Reaktionslösung im klassischen Sinne vorliegt, in der der Katalysator homogen gelöst ist. Hydroformylierungsverfahren, bei denen der Katalysator auf einem Trägermaterial heterogenisiert vorkommt, sind beispielsweise in der WO 2015/028284 A1, der EP 3 632 885 A1, EP 3 744 707 A1, der EP 3 632 886 A1 oder der EP 3 736 258 A1 offenbart.

Bei der industriellen Durchführung derartiger chemischer Reaktionen kann es immer wieder notwendig sein die Anlage z. B. aufgrund von Montage- oder Wartungsarbeiten auch längerfristig abzustellen. Im Rahmen einer solchen Abstellung wird zunächst die Reaktion gestoppt und der Reaktor anschließend mit einem inerten Material, beispielsweise Stickstoff gespült. Dadurch können die im Reaktor vorhandene Produkte ausgetragen und Folgereaktionen der noch vorhandenen Produkte ausgeschlossen werden. Nach einer solchen Abstellprozedur die Reaktion kann es zu Problemen kommen, die das Anfahren der Reaktion unmöglich machen. Grund dafür kann eine stark verminderte Katalysatoraktivität sein, die eine kostspielige Erneuerung des Katalysators erforderlich machen würde.

Die Aufgabe der vorliegenden Erfindung bestand deshalb darin, ein Verfahren zur Hydroformylierung von Olefinen bereitzustellen, welches Schritte zum Abstellen mindestens eines Reaktors umfasst, nicht aber die vorgenannten Nachteile aufweist. Insbesondere sollte sich nach dem Wiederanfahren der Reaktion keine signifikant geringe Katalysatoraktivität ergeben, sondern die Aktivität möglichst erhalten bleiben.

Gelöst wird diese Aufgabe gemäß Anspruch 1 dadurch, dass der mindestens eine Reaktor beim Abstellen mit Synthesegas gespült und bei der anschließenden Lagerung in einer Synthesegasatmosphäre stehen gelassen wird. Bevorzugte Ausgestaltungen sind in den Unteransprüchen angegeben.

Ein Gegenstand der vorliegenden Erfindung ist somit ein Verfahren zur Hydroformylierung von C2-bis C8-Olefinen in mindestens einem Reaktor unter Verwendung eines heterogenisierten Katalysatorsystems, wobei
ein gasförmiges Einsatzgemisch, welches die C2- bis C8-Olefine enthält, zusammen mit Synthesegas in mindestens einem Reaktor über einen in dem mindestens einen Reaktor angeordneten Support aus einem porösen keramischen Material, auf dem das Katalysatorsystem, welches ein Metall aus der 8. oder 9. Gruppe des Periodensystems der Elemente, mindestens einen organischen Phosphor-enthaltenden Liganden, und einen Stabilisator umfasst, heterogenisiert vorliegt, geleitet wird; wobei
der Support ein Monolith, das heißt ein Block aus einem keramischen Material ist, oder in Form eines Pulvers, in Form eines Granulats oder in Form von Formkörpern vorliegt und der Support aus einem carbidischen, nitridischen, silicidischen Material oder Mischungen davon besteht, und wobei die Hydroformylierung bei einer Temperatur in dem mindestens einen Reaktor von 65 bis 200 °C durchgeführt wird; dadurch gekennzeichnet, dass das Verfahren die folgenden Schritte zum Abstellen und Lagern des mindestens einen Reaktors umfasst:
   a) Abstellen der Zufuhr an gasförmigem Einsatzgemisch während weiterhin Synthesegas in den mindestens einen Reaktor zugeführt wird;
   b) Absenken der Temperatur in dem mindestens einen Reaktor auf Umgebungstemperatur;
   c) Abstellen der Zufuhr an Synthesegas und Lagern des mindestens einen Reaktors unter Synthesegasatmosphäre, bis das Verfahren zur Hydroformylierung wieder gestartet wird. Kennzeichnendes Merkmal der vorliegenden Erfindung ist das Spülen des mindestens einen Reaktors mit Synthesegas beim Abstellen der Reaktion und die Lagerung unter Synthesegasatmosphäre. Dadurch lässt sich erreichen, dass es das Verfahren nach dem erneuten Anfahren der Reaktion wieder im Normalbetrieb gefahren werden kann.

Umgebungstemperatur wird in Zusammenhang mit der vorliegenden Erfindung als eine Temperatur verstanden, die ohne Heizen oder Kühlen erreicht wird. Eine genauere Definition ist unmöglich, weil die jeweils vorherrschende Temperatur in Abhängigkeit von z. B. der Jahreszeit signifikant unterschiedlich sein kann.

Im ersten Schritt a) der Abstell- und Lagerschritte wird die Zufuhr an Einsatzgemisch abgestellt. Dadurch wird die Reaktion gestoppt. Gleichzeitig wird der Synthesegasstrom weiterhin dem mindestens einen Reaktor zugeführt. Dadurch können noch im Reaktor befindlicher Kohlenwasserstoffe herausgespült werden. In einer bevorzugten Ausführungsform der vorliegenden Erfindung wird die Synthesegaszufuhr beim Abstellen der Zufuhr an gasförmigem Einsatzgemisch in Schritt a) erhöht. Dadurch kann sichergestellt werden, dass weiterhin ein ähnlicher oder identischer Volumenstrom durch den mindestens einen Reaktor geleitet wird. In eines besonders bevorzugten Ausführungsform wird deshalb die Zufuhr an gasförmigem Einsatzgemisch vollständig durch Synthesegas ersetzt.

Im nachfolgenden Schritt b) wird die Temperatur in dem mindestens einen Reaktor auf Umgebungsdruck abgesenkt. Dies kann aktiv durch Kühlung erfolgen, wobei die Kühlung nur bis zu dem Punkt erfolgt, wo die Umgebungstemperatur in dem mindestens einen Reaktor erreicht wird. Das Abkühlen kann auch passiv, d. h. ohne aktive Kühlung erfolgen. In einer bevorzugten Ausführungsform der vorliegenden Erfindung wird der Druck in dem mindestens einen Reaktor nach dem und/oder während des Absenken(s) der Temperatur in Schritt b) verringert. Dadurch können möglicherweise noch im Reaktor vorhandene Hochsieder entfernt werden, die durch das Spülen in Schritt a) nicht entfernt werden konnten.

In einer weiterhin bevorzugten Ausführungsform der vorliegenden Erfindung kann die Synthesegaszufuhr in Schritt b) verringert werden. Dies kann unter Umständen notwendig sein, um eine Druckverringerung in dem mindestens einen Reaktor erreichen zu können. Weiterhin kann dadurch Synthesegas eingespart werden.

In Schritt c) der Abstell- und Lagerschritte des erfindungsgemäßen Verfahrens wird die Zufuhr an Synthesegas abgestellt und der mindestens eine Reaktor in einer Synthesegasatmosphäre gelagert wird, bis die Hydroformylierung wieder gestartet wird. In einer bevorzugten Ausführungsform kann der Druck vor dem Abstellen der Synthesegaszufuhr in Schritt c) in dem mindestens einen Reaktor wieder erhöht werden. Ist der Druck im dem vorherigen Schritt b) verringert werden, kann die Druckerhöhung notwendig machen.

Das Lagern des mindestens einen Reaktor unter Synthesegasatmosphäre kann über einen bestimmten Zeitraum erfolgen, beispielsweise für Tage, Wochen, Monate oder Jahre. In einer bevorzugten Ausführungsform wird der mindestens eine Reaktor für mindestens 24 Stunden unter Synthesegasatmosphäre gelagert.

Als Einsatzgemisch können alle Gemische eingesetzt werden, die C2- bis C8-Olefine, vorzugsweise C2- bis C5-Olefine, insbesondere Ethen, Propen, 1-Buten, 2-Buten, 1-Penten oder 2-Penten, als Edukte umfassen. Die Menge an Olefinen in den Einsatzgemischen sollte verständlicherweise hoch genug sein, um eine Hydroformylierungsreaktion wirtschaftlich betreiben zu können. Zu den in dem erfindungsgemäßen Verfahren einsetzbaren Einsatzgemischen gehören insbesondere auch technische Gemische der petrochemischen Industrie, wie beispielsweise Raffinatströme (Raffinat I, II oder III) oder Rohbutan. Rohbutan umfasst gemäß der vorliegenden Erfindung 5 bis 40 Gew.-% Butene, vorzugsweise 20 bis 40 Gew.-% Butene (die Butene setzen sich zusammen aus 1 bis 20 Gew.-% 1-Buten und 80 bis 99 Gew.-% 2-Buten) und 60 bis 95 Gew.- % Butane, vorzugsweise 60 bis 80 Gew.-% Butane.

Das erfindungsgemäße Verfahren wird in mindestens einem Reaktor, in dem die erfindungsgemäße Hydroformylierung stattfindet, durchgeführt. In dem mindestens einen Reaktor ist der Support mit dem heterogenisierten Katalysatorsystem angeordnet. In einer weiteren Ausführungsform der vorliegenden Erfindung kann das Verfahren auch in mehrere Reaktoren, die parallel oder in Reihe geschaltet vorliegen können, durchgeführt werden. Sind mehrere Reaktoren vorhanden, sind diese Reaktoren vorzugsweise parallel geschaltet und werden alternierend verwendet.

Die Hydroformylierung wird vorzugsweise bei den folgenden Bedingungen durchgeführt: Die Temperatur bei der Hydroformylierung liegt im Bereich von 65 bis 200 °C, vorzugsweise 75 bis 175 °C und besonders bevorzugt 85 bis 150 °C. Die Temperatur kann über einen geeignete Kühlvorrichtung, beispielsweise einen Kühlmantel eingestellt werden. Der Druck während der Hydroformylierung sollte mindestens 1 bar betragen und nicht größer als 35 bar, vorzugsweise nicht größer als 30 bar, besonders bevorzugt nicht größer als 25 bar sein. Das molare Verhältnis zwischen Synthesegas und dem Einsatzgemisch sollte zwischen 6:1 und 1:1, vorzugsweise zwischen 5:1 und 3:1 liegen. Optional kann das Einsatzgemisch mit Inertgas verdünnt werden, beispielsweise mit den in technischen Kohlenwasserstoffströmen befindlichen Alkanen.

Das bei dem erfindungsgemäßen Hydroformylierungsverfahren eingesetzte Katalysatorsystem umfasst vorzugsweise ein Übergangsmetall aus der 8. oder 9. Gruppe des Periodensystems der Elemente, insbesondere Eisen, Ruthenium, Iridium, Cobalt oder Rhodium, weiterhin bevorzugt Cobalt und Rhodium, besonders bevorzugt Rhodium, mindestens einen organischen Phosphor-enthaltenden Liganden und einen Stabilisator.

Der Stabilisator ist vorzugsweise eine organische Aminverbindung, besonders bevorzugt eine organische Aminverbindung, die mindestens eine 2,2,6,6-Tetramethylpiperidineinheit nach Formel (I) enthält:

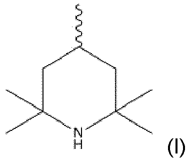

In einer besonders bevorzugten Ausführungsform der vorliegenden Erfindung ist der Stabilisator ausgewählt aus der Gruppe, bestehend aus den Verbindungen der nachfolgenden Formeln (I.1), (I.2), (I.3), (I.4), (I.5), (I.6), (I.7) und (I.8). 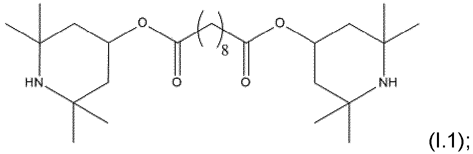 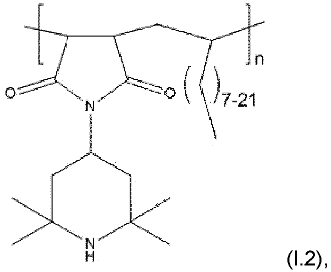
wobei n einer ganzen Zahl von 1 bis 20 entspricht; 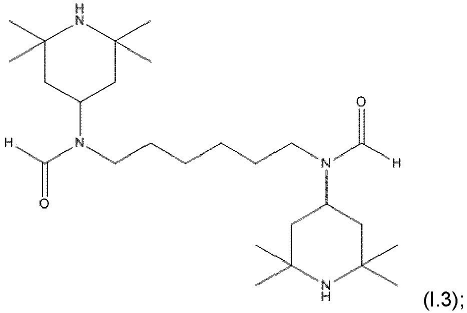 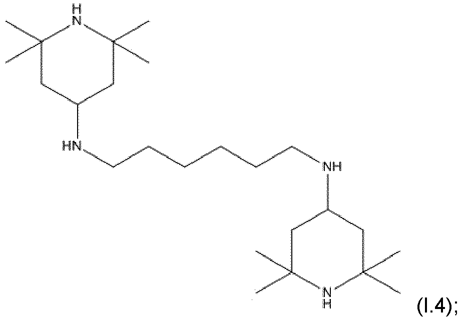 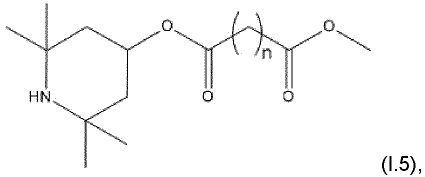
wobei n einer ganzen Zahl von 1 bis 12 entspricht; 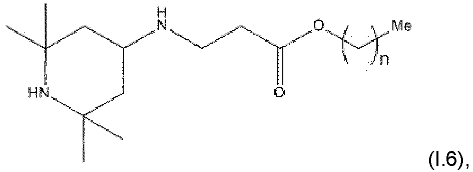
wobei n einer ganzen Zahl von 1 bis 17 entspricht; 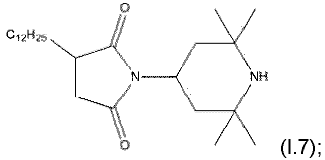 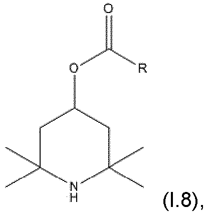
wobei R einer C6- bis C20-Alkylgruppe entspricht.

Für alle filmbildenden Komponenten, d. h. in diesem Fall der Stabilisator, sollte die Gaslöslichkeit für die Reaktanden besser sein als die Gaslöslichkeit der Produkte. Bereits dadurch kann eine partielle Stofftrennung zwischen eingesetzten Eduktolefinen und gebildeten Produktaldehyden erreicht werden. Grundsätzlich wären dafür auch andere filmbildende Substanzen denkbar, allerdings ist darauf zu achten, dass es nicht zu einer erhöhten Hochsiederbildung kommt und/oder die Nachführung der Eduktolefine eingeschränkt wird.

Der organische Phosphor-enthaltende Ligand für das erfindungsgemäße Katalysatorsystem kann aus den für die Hydroformylierungen bekannten Liganden ausgewählt werden. Eine Vielzahl geeigneter Liganden ist dem Fachmann aus der Patent- und Fachliteratur bekannt, beispielsweise Mono- oder Biphosphitliganden. Der organische Phosphor-enthaltende Ligand weist vorzugsweise gemäß der allgemeinen Formel (II) eine Biphosphitstruktur auf

R'-A-R"-A-R‴ (II)

wobei R', R" und R‴ jeweils organische Reste sind und beide A jeweils eine brückende -O-P(-O)₂-Gruppe sind, wobei zwei der drei Sauerstoffatome -O- jeweils an Rest R' und den Rest R‴ gebunden sind, mit der Maßgabe das R' und R‴ nicht identisch sind. Die organischen Reste R', R" und R‴ enthalten vorzugsweise keine endständige Trialkoxysilangruppe.

In einer bevorzugten Ausführungsform sind R', R" und R‴ in der Verbindung der Formel (VI), vorzugsweise ausgewählt aus substituierten oder unsubstituierten 1,1'-Biphenyl-, 1,1'-Binaphthyl- und ortho-Phenylgruppen, insbesondere aus substituierten oder unsubstituierten 1,1'-Biphenylgruppen, mit der Maßgabe das R' und R‴ nicht identisch sind. Besonders bevorzugt weisen die substituierten 1,1'-Biphenylgruppen in 3,3'- und/oder 5,5'-Stellung des 1,1'-Biphenylgrundkörpers eine Alkylgruppe und/oder eine Alkoxygruppe auf, insbesondere eine C1-C4-Alkylgruppe, besonders bevorzugt eine tert.-Butyl- und/oder Methylgruppe und/oder bevorzugt einen C1-C5-Alkoxgruppe, besonders bevorzugt eine Methoxygruppe.

Das vorgenannte Katalysatorsystem liegt erfindungsgemäß heterogenisiert auf dem Support aus einem porösen keramischen Material vor. Im Sinne der vorliegenden Erfindung ist der Ausdruck "heterogenisiert auf einem Support" so zu verstehen, dass das Katalysatorsystem durch Ausbildung eines dünnen, festen oder flüssigen Films mithilfe des Stabilisators auf der inneren und/oder äußeren Oberfläche des Supports immobilisiert wird. Der Film kann auch bei Raumtemperatur fest und bei Reaktionsbedingungen flüssig sein.

Die innere Oberfläche des festen Trägermaterials umfasst insbesondere die innere Oberfläche der Poren. Immobilisierung umfasst begrifflich sowohl den Fall, dass das Katalysatorsystem und/oder die katalytisch aktive Spezies gelöst in dem festen oder flüssigen Film vorliegen, als auch die Fälle, dass der Stabilisator als Haftvermittler wirkt oder dass das Katalysatorsystem auf der Oberfläche adsorbiert wird, nicht jedoch chemisch bzw. kovalent gebunden auf der Oberfläche vorliegt.

Es liegt erfindungsgemäß also keine Reaktionslösung im klassischen Sinne vor, in der der Katalysator homogen gelöst ist, sondern das Katalysatorsystem befindet sich dispers verteilt auf der Oberfläche und/oder in den Poren des Supports.

Das poröse Supportmaterial ist vorzugsweise ausgewählt aus der Gruppe, bestehend aus einer nitridischen Keramik, einer carbidischen Keramik, einer silicidischen Keramik und Mischungen davon, beispielsweise carbonitridische Materialien.

Die nitridische Keramik ist vorzugsweise ausgewählt aus Siliciumnitrid, Bornitrid, Aluminiumnitrid und Mischungen davon. Die carbidische Keramik ist vorzugsweise ausgewählt aus Siliciumcarbid, Borcarbid, Wolframcarbid oder Mischungen davon. Denkbar sind auch Mischungen aus carbidischen und nitridischen Keramik, die sogenannten Carbonitride. Die silicidische Keramik ist vorzugsweise Molybdändisilicid. Der Support gemäß der vorliegenden Erfindung, auf den das Katalysatorsystem aufgebracht wird, besteht vorzugsweise aus einer carbidischen Keramik, besonders bevorzugt aus Siliciumcarbid.

Der Support kann im vorliegenden Fall als Monolith, das heißt einem Block aus einem keramischen Material, oder in Form eines Pulvers, in Form eines Granulats oder in Form von Formkörpern vorliegen.

Ist der Support ein Monolith, besteht der Support aus einem Block (ein dreidimensionales Objekt) aus dem porösen keramischen Material. Der Block kann sowohl einstückig ausgebildet sein als auch aus mehreren, also mindestens zwei Einzelteilen bestehen, die zu dem Block zusammengefügt werden können und/oder miteinander fest oder lösbar verbunden sind.

Der Support aus dem porösen keramischen Material ist vorzugsweise ein sich dreidimensional erstreckendes Bauteil, welches in seinem Querschnitt grundsätzlich beliebige geometrische Formen, beispielsweise rund, eckig, quadratisch o. ä., aufweisen kann. Das sich dreidimensional erstreckende Bauteil, welches als Support eingesetzt werden kann, weist in einer bevorzugten Ausführungsform eine Längsrichtung (Richtung der längsten Ausdehnung) in Hauptdurchströmungsrichtung (Richtung in die das Einsatzgemisch und das Synthesegas von Einlass zum Auslass des mindestens einen Reaktors strömen) auf.

Der so geformte Supportmonolith aus dem porösen keramischen Material weist zumindest einen durchgängigen Kanal in Hauptdurchströmungsrichtung auf. Der oder die Kanäle können jedoch auch so ausgestaltet sein, dass sie nicht komplett durchgängig sind, sondern zu dem Ende, welches dem Einlass des mindestens einen Reaktors gegenüberliegt, einen Abschluss aufweisen bzw. der Kanal zu diesem Ende hin geschlossen ist. Der Supportmonolith kann auch mindestens zwei oder mehr Kanäle aufweisen. Der Durchmesser der Kanäle kann im Bereich von 0,25 bis 50 mm, vorzugsweise im Bereich von 1 bis 30 mm, weiterhin bevorzugt im Bereich von 1,5 bis 20 mm und besonders bevorzugt im Bereich von 2 bis16 mm liegen. Sind mehrere Kanäle vorhanden, können die Durchmesser der Kanäle gleich oder verschieden voneinander sein. Der Durchmesser der Kanäle ist im Vergleich zu dem oder zu einem der Durchmesser des gesamten Supports insbesondere so zu wählen, dass die mechanische Stabilität nicht beeinträchtigt wird.

Darüber hinaus ist der Supportmonolith aus dem keramischen Material porös, weist also Poren auf. Das erfindungsgemäße Katalysatorsystem befindet sich insbesondere auch in dem festen oder flüssigen Film in diesen Poren. Der Porendurchmesser liegt vorzugsweise im Bereich von 0,9 nm bis 30 µm, vorzugsweise im Bereich von 10 nm bis 25 µm und besonders bevorzugt im Bereich von 70 nm bis 20 µm. Der Porendurchmesser kann mittels Stickstoffadsorption oder Quecksilber-Porosimetrie gemäß DIN 66133 (Stand: 1993-06) bestimmt werden.

In einer bevorzugten Ausführungsform weist der Supportmonolith zumindest teilweise durchgängige Poren auf, die sich von der Oberfläche zu den Kanälen hin und/oder von einem Kanal zu dem oder zu den nächstliegenden Kanälen erstrecken. Möglich ist auch das mehrere Poren miteinander verbunden sind und so insgesamt eine einzige durchgängige Pore bilden.

Der Support kann erfindungsgemäß auch in Form eines Pulvers, in Form eines Granulats oder in Form von Formkörpern wie Pellets, Ringe, Kugeln o.ä. vorliegen. Der mittlere Partikeldurchmesser (d50) des Supports kann von 0,1 mm bis 7 mm, vorzugsweise 0,3 bis 6 mm, besonders bevorzugt von 0,5 mm bis 5 mm betragen. Der mittlere Partikeldurchmesser kann mittels bildgebender Verfahren ermittelt werden, insbesondere durch die in den Normen ISO 13322-1 (Stand: 2004-12-01) und ISO 13322-2 (Stand: 2006-11-01) genannten Verfahren ermittelt werden. Die Herstellung des Supports in Form eines Pulvers, in Form eines Granulats oder in Form von Formkörpern kann nach dem Fachmann bekannten Verfahren erfolgen. Beispielsweise könnte es dadurch erfolgen, dass ein Monolith aus dem carbidischen, nitridischen, silicidischen Material oder Mischungen davon mechanisch zerkleinert wird, beispielsweise mit einem Backenbrecher, und die Partikelgröße des erhaltenen Bruchgranulats mittels Sieben eingestellt wird.

Enstsprechend dem Supportmonolith sind die Partikel des Pulvers, des Granulats oder die Formkörper aus dem keramischen Material porös, weisen also Poren auf. Das erfindungsgemäße Katalysatorsystem befindet sich insbesondere auch in dem festen oder flüssigen Film in diesen Poren. Der Porendurchmesser liegt vorzugsweise im Bereich von 0,9 nm bis 30 µm, vorzugsweise im Bereich von 10 nm bis 25 µm und besonders bevorzugt im Bereich von 70 nm bis 20 µm. Der Porendurchmesser kann mittels Stickstoffadsorption oder Quecksilber-Porosimetrie gemäß DIN 66133 (Stand: 1993-06) bestimmt werden.

Die Herstellung des Supports, egal ob Monolith, Pulver, Granulat oder Formkörper, erfolgt wie nachfolgend beschrieben:
Auf den bereitgestellten pulver-, granulat- oder pelletförmigen Support aus dem keramischen Material kann zusätzlich ein sogenannter Washcoat aufgetragen werden, welcher bezogen auf das keramische Material des Supports aus dem gleichen oder einem unterschiedlichen Keramikmaterial, vorzugsweise Siliciumoxid. Der Washcoat selbst kann porös oder unporös sein, vorzugsweise ist der Waschcoat unporös. Die Partikelgröße des Washcoats beträgt vorzugsweise 5 nm bis 3 µm, vorzugsweise 7 nm bis 700nm. Der Washcoat wird verwendet, um die gewünschte Porengröße einzubringen oder zu generieren und/oder, um die Oberfläche des Supports zu erhöhen. Der Auftrag des Washcoats kann insbesondere mittels Eintauchen (Dipcoating) in eine Washcoat-Lösung, die das Keramikmaterial des Washcoats, ggf. auch als Precursor, enthält, erfolgen. Die Menge das auf dem Support befindlichen Waschcoats beträgt ≤ 20 Gew.-%, vorzugsweise ≤ 15 Gew.-%, besonders bevorzugt ≤ 10 Gew.-% bezogen auf die Gesamtmenge des Supports. In einer bevorzugten Ausführungsform der vorliegenden Erfindung weist der Support aber keinen Washcoat auf.

Auf den Support mit oder ohne Washcoat wird das Katalysatorsystem aufgebracht. Dazu wird zunächst eine Katalysatorlösung durch Mischen, insbesondere bei Raumtemperatur und Umgebungsdruck, hergestellt, wobei die Katalysatorlösung mindestens einen organischen Phosphor-haltigen Ligand, mindestens einen Metall-Precursor, beispielsweise Chloride, Oxide, Carboxylate des jeweiligen Metalls, mindestens einen Stabilisator und mindestens ein Lösemittel umfasst. Optional kann bei der Herstellung des Katalysatorsystems eine ionische Flüssigkeit verwendet werden, die Katalysatorlösung kann aber auch explizit ohne ionische Flüssigkeit angesetzt werden. Die Herstellung der Katalysatorlösung sollte insbesondere in einer inerten Umgebung, bspw. einer Glovebox erfolgen. Inerte Umgebung heißt in diesem Fall eine möglichst Wasser- und Sauerstoff-freie Atmosphäre.

Das Lösemittel kann aus allen Lösemittelklassen gewählt werden (protisch, aprotisch, polar oder unpolar) Voraussetzung für das Lösemittel ist die Löslichkeit von Katalysatorsystem (Ligand, Metall Precursor, Stabilisator und optional die ionische Flüssigkeit) und bevorzugt auch der bei der Hydroformylierung entstehenden Hochsieder. Die Löslichkeit kann innerhalb des Immobilisierungsschrittes durch Aufheizen erhöht werden.

Das Lösemittel ist vorzugsweise aprotisch, polar, wie z. B. Acetonitril und Ethylacetat oder aber auch aprotisch, unpolar wie z. B. THF und Dietehylether. Auch Chlorkohlenwasserstoffe wie z. B. Dichlormethan oder Aldehyde können als Lösemittel verwendet werden.

Die so hergestellte Katalysatorlösung wird dann mit dem Support (optional inkl. Washcoat) in Kontakt gebracht, beispielsweise durch Eintauchen (Dip-Coating) oder durch Befüllen eines Druckgefäßes, beispielsweise direkt in dem mindestens einen Reaktor (in-situ-Imprägnierung). Sofern das Aufbringen der Katalysatorlösung außerhalb des Reaktors erfolgt, muss der Support nach Abtrennen des Lösemittels natürlich wieder in den Reaktor eingebaut werden. Bevorzugt wird die Katalysatorlösung direkt im Reaktor auf den Support mit dem Washcoat aufgebracht, weil dadurch möglicherweise zeitaufwändige Ein- und Ausbauschritte sowie eine mögliche Kontamination des Katalysators vermieden werden können.

Das Befüllen des Reaktors mit der Katalysatorlösung kann über die normalen Ein- bzw. Ausgänge erfolgen, beispielsweise mittels einer Pumpe. Flüssigkeitsverteiler oder Düsen innerhalb des Reaktors können für eine gleichmäßige Verteilung der Katalysatorflüssigkeit sorgen, ebenso wie optional vorliegende Druckverlusteinbauten oder Regelungen für die Dosiergeschwindigkeit.

Nach dem Aufbringen des Katalysatorsystems wird das Lösemittel abgetrennt. Dabei wird zunächst die restliche Katalysatorlösung über den Auslass des Reaktors abgelassen. Danach werden im Reaktor verbliebene Lösemittelreste durch Einstellen des Drucks oder Erhöhung der Temperatur verdampft. Die Einstellung des Drucks kann in einer anderen Ausführungsform auch unter gleichzeitiger Erhöhung der Temperatur durchgeführt werden. Die Temperatur kann in Abhängigkeit vom Lösemittel 20 bis 150 °C betragen. Der Druck kann in Abhängigkeit vom Lösemittel auf ein Hochvakuum (10⁻³ bis 10⁻⁷ mbar) eingestellt werden, je nach Lösemittel und Temperatur sind aber auch Überdrücke von einigen mbar bis zu mehreren bar denkbar.

Der Stabilisator und die optional vorhandene ionische Flüssigkeit verbleiben mit dem Katalysator aus dem Übergangsmetall, insbesondere Cobalt oder Rhodium, und dem organischen Phosphor-haltigen Liganden heterogenisiert auf dem Support.

Das Aufbringen des Katalysatorsystem auf den Support kann sowohl direkt im Reaktor (in situ) oder außerhalb des Reaktors erfolgen. Wird das Katalysatorsystem außerhalb des Reaktors aufgebracht, sollte der Support immer unter Luftabschluss transportiert werden muss, was beispielsweise mit einem Stickstoffgegenstrom realisiert werden. Das Aufbringen des Katalysatorsystems wird in einer bevorzugten Ausführungsform der vorliegenden Erfindung direkt im Reaktor, also in situ, aufgebracht. Nach dem Abtrennen des Lösemittels kann der Reaktor sofort eingesetzt werden und mit dem Einsatzgemisch beschickt werden. Das hat den Vorteil, dass keine zeitaufwändigen Ein- und Ausbauschritte notwendig sind, die einen längeren Ausfall des Reaktors zur Folge hätten. Zudem ist die Größe des Supports dann nicht mehr dadurch limitiert, dass geeignete Räumlichkeiten mit inerten Umgebungen einer bestimmten Größe vorliegen. Die Größe des Supports kann in Abhängigkeit vom Reaktordesign frei gewählt werden.

Nach erfolgtem Aufbringen des Katalysatorsystems auf den Support und erfolgter Abtrennung des Lösemittels kann die Anlage, insbesondere der Reaktor durch eine zwei- oder mehrstufige Anfahrprozedur hochgefahren werden, also in den Betrieb überführt werden. Eine geeignete Anfahrprozedur ist beispielsweise in der EP 3 632 887 beschrieben.

Aus der Reaktionszone, in der die erfindungsgemäße Hydroformylierung durchgeführt wird, wird vorzugsweise kontinuierlich ein gasförmiger Austrag entnommen, der mindestens einen Teil der entstandenen Produktaldehyde und mindestens einen Teil der nicht umgesetzten Olefine enthält. Der gasförmige Austrag kann einem oder mehreren Stofftrennungsschritt(en) unterworfen werden, bei der der gasförmige Austrag in mindestens eine an nicht umgesetzten Olefinen-reiche Phase und mindestens eine Produktaldehyd-reiche Phase getrennt wird.

Die Stofftrennnung kann mit bekannten Stofftrennungsverfahren, wie Kondensation, Destillation, Zentrifugation, Nanofiltration oder Kombination von mehreren davon, vorzugsweise Kondensation oder Destillation, durchgeführt werden.

Im Falle einer mehrstufigen Stofftrennung kann die bei der ersten Stofftrennung gebildete Produktaldehyd-reiche Phase einer zweiten Stofftrennung, insbesondere einer nachfolgenden Aldehydabtrennung zugeführt werden, bei der das Produktaldehyd von den anderen in dieser Phase befindlichen Stoffen, häufig Alkane und Eduktolefine, abgetrennt wird. Die an nicht umgesetzten Olefin-reiche Phase kann zu dem Hydroformylierungsschritt oder in Falle einer mehrstufigen Ausgestaltung zu einer der Hydroformylierungsschritten zurückgeführt werden, um die darin enthaltenen Olefine auch noch zum Produktaldehyd zu hydroformylieren.

Bei der Stofftrennung kann neben den genannten Phasen auch ein Purgegasstrom entnommen werden, der eine zur an nicht umgesetzten Olefin-reiche Phase zumindest ähnliche oder identische Zusammensetzung aufweist. Der Purgegasstrom kann ebenfalls zur zweiten Stofftrennung bzw. Aldehydabtrennung geleitet werden, um die darin enthaltenen Produktaldehyde abzutrennen und um Verunreinigungen (z. B. Stickstoff im Synthesegas) oder inerte Substanzen (z. B. Alkane im Einsatzgemisch) aus dem System auszutragen. Die Verunreinigungen oder inerte Substanzen können üblicherweise bei der zweiten Stofftrennung als leichtflüchtige Stoffe, beispielsweise am Kopf einer Kolonne, abgenommen werden.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert.

Beispiel 1 (Vergleichsbeispiel) Als Ausgangsmaterial für den Support wurden SiC-Pellets verwendet (SIKAT SarL), Die SiC-Pellets wurden in eine 20 cm lange runde Reaktorhülse mit einem Durchmesser von einem Zoll (ca. 2,54 cm) eingebracht, wobei ober- und unterhalb des Granulats Glasperlen ähnlicher Größe eingefüllt wurden. Die SiC-Pellets wurde dann mit einer Katalysatorlösung, enthaltend Rh(acac)(CO)₂, Bisphephos (Ligand), Bis(2,2,6,6-tetramethyl-4-piperidyl)sebacat (Stabilisator) und Pentanal als Lösemittel beaufschlagt. Dazu wurde die Katalysatorlösung nach dem Spülen des Reaktors mit Stickstoff mit leichtem Überdruck in den Reaktor eingeleitet. Nach dem Entfernen des Lösemittels aus dem Reaktor durch Ablassen und Verdampfen wurde das auf dem Supportgranulat heterogenisierte Katalysatorsystem zur Hydroformylierung verwendet.

Als Einsatzgemisch für die Versuche wurde ein Kohlenwasserstoffstrom mit 15 Gew.-% Butenen und 85 Gew.-% Butanen eingesetzt. Das Einsatzgemisch wurde zusammen mit Synthesegas (molares Verhältnis Synthesegas : Einsatzgemisch = 3,5 : 1) zur Hydroformylierung mit einem Gasvolumenstrom von 390 ml/min in den Reaktor geleitet. Die Hydroformylierung wurde bei einer Temperatur von 120 - 130 °C und einem Druck von 17 bar durchgeführt.

Während des Versuchs wurde der Strom der Reaktanden für mehrere längere Zeiträume unterbrochen (Standzeit) und in dieser Standzeit wurde der weiterhin auf 120 °C erwärmte Reaktor mit einem Stickstoffstrom bei einem Druck von 17 bar gespült. Umsatz und Ausbeute wurden jeweils vor und nach der Standzeit mittels Gaschromatographie vom Ausgangsstrom bestimmt.

**Tabelle 1: Ergebnisse von Beispiel 1**

| Beispiel 1 | Ausbeute | Umsatz |
|---|---|---|
| Vorher | 24,5% | 45,8% |
| nachher | 11% | 30,8% |

### Beispiel 2

Als Ausgangsmaterial für den Support wurden SiC-Pellets verwendet (SIKAT SarL), Die SiC-Pellets wurden in eine 20 cm lange runde Reaktorhülse mit einem Durchmesser von einem Zoll (ca. 2,54 cm) eingebracht, wobei ober- und unterhalb des Granulats Glasperlen ähnlicher Größe eingefüllt wurden. Die SiC-Pellets wurde dann mit einer Katalysatorlösung, enthaltend Rh(acac)(CO)₂, Bisphephos (Ligand), Bis(2,2,6,6-tetramethyl-4-piperidyl)sebacat (Stabilisator) und Pentanal als Lösemittel beaufschlagt. Dazu wurde die Katalysatorlösung nach dem Spülen des Reaktors mit Stickstoff mit leichtem Überdruck in den Reaktor eingeleitet. Nach dem Entfernen des Lösemittels aus dem Reaktor durch Ablassen und Verdampfen wurde das auf dem Supportgranulat heterogenisierte Katalysatorsystem zur Hydroformylierung verwendet.

Als Einsatzgemisch für die Versuche wurde ein Kohlenwasserstoffstrom mit 15 Gew.-% Butenen und 85 Gew.-% Butanen eingesetzt. Das Einsatzgemisch wurde zusammen mit Synthesegas (molares Verhältnis Synthesegas : Einsatzgemisch = 3,5 : 1) zur Hydroformylierung mit einem Gasvolumenstrom von 390 ml/min in den Reaktor geleitet. Die Hydroformylierung wurde bei einer Temperatur von 120 - 130 °C und einem Druck von 17 bar durchgeführt.

Während des Versuchs wurde eine Abstellung des Reaktors simuliert. Hierfür wurde der Strom der Reaktanden für längere Zeiträume unterbrochen, der Reaktor auf Umgebungstemperatur abgekühlt und nach einer Standzeit von 23 Tagen wieder angefahren. Bei der Lagerung wurde der Reaktor währenddessen mit Synthesegas beaufschlagt.

Im ersten Schritt wurde der Fluss des Kohlenwasserstoffstroms gestoppt und zumindest partiell durch Synthesegas ersetzt. Nach einer längeren Spülphase nahe der Reaktionstemperatur wurde der Druck im Reaktor abgesenkt, um weitere Produktreste auszutreiben. Hiernach wurde das System auf Raumtemperatur abgekühlt und letztlich zur Lagerung mit Synthesegas auf Reaktionsdruck aufgedrückt und das System abgeschlossen.

Nach einer Standzeit von 23 Tagen wurde das System wieder angefahren. Zum Anfahren wurde der Reaktor zunächst wieder mit Synthesegas durchspült, auf 10bar aufgedrückt und auf Reaktionstemperatur 120°C aufgeheizt. Die Zudosierung des Kohlenwasserstoffstroms wurde in mehreren Stufen durchgeführt (wie in EP 3 632 887 A1 beschrieben). Vor und nach der Abstellung und Lagerung wurde die Reaktorperformance anhand des Umsatzes, der Ausbeute und der Selektivität mittels Gaschromatographie vom Ausgangsstrom bestimmt.

**Tabelle 2: Ergebnisse des Beispiels 2**

| Beispiel 2 | Umsatz (Butene gesamt) | n/iso Selektivität | Ausbeute |
|---|---|---|---|
| Vor Abstellung | 17% | 96% | 15% |
| Nach Abstellung und Lagerung | 18% | 96% | 15% |

Es zeigt sich, dass nach dem erfindungsgemäßen Abstellen und Lagern Umsatz, Selektivität und Ausbeute nahezu identisch sind zu den Werten vor der Abstellung. Die Katalysatoraktivität ist folglich nahezu konstant geblieben und nicht wie in Beispiel 1 stark abgesunken, obwohl in Beispiel 1 sogar nur mit Stickstoff gespült worden ist.

## Patentansprüche

1. Verfahren zur Hydroformylierung von C2- bis C8-Olefinen in mindestens einem Reaktor unter Verwendung eines heterogenisierten Katalysatorsystems, wobei ein gasförmiges Einsatzgemisch, welches die C2- bis C8-Olefine enthält, zusammen mit Synthesegas in mindestens einem Reaktor über einen in dem mindestens einen Reaktor angeordneten Support aus einem porösen keramischen Material, auf dem das Katalysatorsystem, welches ein Metall aus der 8. oder 9. Gruppe des Periodensystems der Elemente, mindestens einen organischen Phosphor-enthaltenden Liganden, einen Stabilisator umfasst, heterogenisiert vorliegt, geleitet wird; wobei
der Support ein Monolith, das heißt ein Block aus einem keramischen Material ist, oder in Form eines Pulvers, in Form eines Granulats oder in Form von Formkörpern vorliegt und der Support aus einem carbidischen, nitridischen, silicidischen Material oder Mischungen davon besteht, und wobei
die Hydroformylierung bei einer Temperatur im Reaktor von 65 bis 200 °C durchgeführt wird; **dadurch gekennzeichnet, dass** das Verfahren die folgenden Schritte zum Abstellen des mindestens einen Reaktors umfasst:
a) Abstellen der Zufuhr an gasförmigem Einsatzgemisch während weiterhin Synthesegas in den mindestens einen Reaktor zugeführt wird;
b) Absenken der Temperatur in dem mindestens einen Reaktor auf Umgebungstemperatur;
c) Abstellen der Zufuhr an Synthesegas und Lagern des mindestens einen Reaktors unter Synthesegasatmosphäre, bis das Verfahren zur Hydroformylierung wieder gestartet wird.

2. Verfahren nach Anspruch 1, wobei beim Abstellen der Zufuhr an gasförmigem Einsatzgemisch in Schritt a) die Synthesegaszufuhr erhöht wird.

3. Verfahren nach Anspruch 1 oder 2, wobei der Druck in dem mindestens einen Reaktor nach dem und/oder während des Absenken(s) der Temperatur in Schritt b) verringert wird.

4. Verfahren nach Anspruch 3, wobei die Synthesegaszufuhr bereits in Schritt b) verringert wird.

5. Verfahren nach einem der vorherigen Ansprüche, wobei vor dem Abstellen der Synthesegaszufuhr in Schritt c) der Druck im Reaktor wieder erhöht wird.

6. Verfahren nach einem der vorhergehenden Ansprüche, wobei der organische Phosphor-enthaltende Ligand des Hydroformylierungskatalysatorsystems vorzugsweise die allgemeine Formel (II)
R'-A-R"-A-R‴ (II)
aufweist, wobei R', R" und R‴ jeweils organische Reste sind, mit der Maßgabe das R' und R‴ nicht identisch sind, und beide A jeweils eine brückende -O-P(-O)₂-Gruppe sind, wobei zwei der drei Sauerstoffatome -O- jeweils an Rest R' und den Rest R‴ gebunden sind.

7. Verfahren nach einem der vorhergehenden Ansprüche, wobei der Stabilisator eine organische Aminverbindung ist, die mindestens eine 2,2,6,6-Tetramethylpiperidineinheit nach Formel (I) enthält:

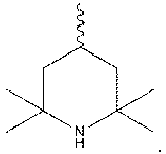

8. Verfahren nach einem der vorhergehenden Ansprüche, wobei die nitridische Keramik ausgewählt wird aus Siliciumnitrid, Bornitrid, Aluminiumnitrid und Mischungen davon; die carbidische Keramik ausgewählt wird aus Siliciumcarbid, Borcarbid, Wolframcarbid oder Mischungen davon; und die silicidische Keramik Molbydänsilicid ist.

9. Verfahren nach Anspruch 8, wobei der Support aus einer carbidische Keramik besteht.

10. Verfahren nach Anspruch 9, wobei der Support aus Siliciumcarbid besteht.

11. Verfahren nach einem der Ansprüche 1 bis 7, wobei die Hydroformylierung bei einer Temperatur im Bereich von 75 bis 175 °C, vorzugsweise im Bereich von 85 bis 150 °C, durchgeführt wird.

12. Verfahren nach einem der vorhergehenden Ansprüche, wobei der Druck bei der Hydroformylierung mindestens 1 bar und nicht größer als 35 bar, vorzugsweise nicht größer als 30 bar, besonders bevorzugt nicht größer als 25 bar ist.

13. Verfahren nach einem der vorhergehenden Ansprüche, wobei das Katalysatorsystem keine ionische Flüssigkeit umfasst.

14. Verfahren nach einem der vorhergehenden Ansprüche, wobei bei dem Verfahren zur Hydroformylierung C4-Olefine eingesetzt werden.

15. Verfahren nach einem der vorhergehenden Ansprüche, wobei als ein Metall aus der 8. oder 9. Gruppe des Periodensystems der Elemente Rhodium eingesetzt wird.

## Claims

1. Process for hydroformylating C2 to C8 olefins in at least one reactor using a heterogenized catalyst system, wherein
a gaseous feed mixture containing the C2 to C8 olefins is passed together with synthesis gas in at least one reactor over a support which is arranged in the at least one reactor and is composed of a porous ceramic material on which the catalyst system, which comprises a metal from group 8 or 9 of the periodic table of the elements, at least one organic phosphorus-containing ligand and a stabilizer, is present in heterogenized form; wherein the support is a monolith, i.e. a block of a ceramic material, or is in the form of a powder, in the form of a granular material or in the form of shaped bodies and the support consists of a carbidic material, nitridic material, silicidic material or mixtures thereof, and wherein
the hydroformylation is conducted at a temperature in the reactor of 65 to 200°C;
**characterized in that** the process comprises the following steps for shutdown of the at least one reactor:
a) shutting down the feed of gaseous feed mixture while synthesis gas is still being fed into the at least one reactor;
b) lowering the temperature in the at least one reactor to ambient temperature;
c) shutting down the feed of synthesis gas and keeping the at least one reactor under synthesis gas atmosphere until the hydroformylation process is restarted.

2. Process according to Claim 1, wherein the synthesis gas feed rate is increased when the feed of gaseous feed mixture is shut down in step a).

3. Process according to Claim 1 or 2, wherein the pressure in the at least one reactor is reduced after and/or during the lowering of the temperature in step b).

4. Process according to Claim 3, wherein the synthesis gas feed rate is already reduced in step b).

5. Process according to any of the preceding claims, wherein, before the synthesis gas feed is shut down in step c), the pressure in the reactor is increased again.

6. Process according to any of the preceding claims, wherein the organic phosphorus-containing ligand of the hydroformylation catalyst system preferably has the general formula (II)
R'-A-R''-A-R‴ (II)
where R', R'' and R‴ are each organic radicals, with the proviso that R' and R‴ are nonidentical, and the two A are each a bridging -O-P(-O)₂- group, wherein two of the three oxygen atoms -O- are attached respectively to radical R' and to the radical R‴.

7. Process according to any of the preceding claims, wherein the stabilizer is an organic amine compound containing at least one 2,2,6,6-tetramethylpiperidine unit of formula (I):

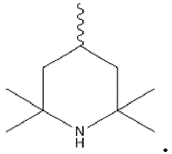

8. Process according to any of the preceding claims, wherein the nitridic ceramic is selected from silicon nitride, boron nitride, aluminium nitride and mixtures thereof; the carbidic ceramic is selected from silicon carbide, boron carbide, tungsten carbide or mixtures thereof; and the silicidic ceramic is molybdenum silicide.

9. Process according to Claim 8, wherein the support consists of a carbidic ceramic.

10. Process according to Claim 9, wherein the support consists of silicon carbide.

11. Process according to any of Claims 1 to 7, wherein the hydroformylation is conducted at a temperature in the range from 75 to 175°C, preferably in the range from 85 to 150°C.

12. Process according to any of the preceding claims, wherein the pressure in the hydroformylation is at least 1 bar and not greater than 35 bar, preferably not greater than 30 bar, more preferably not greater than 25 bar.

13. Process according to any of the preceding claims, wherein the catalyst system does not comprise an ionic liquid.

14. Process according to any of the preceding claims, wherein C4 olefins are used in the hydroformylation process.

15. Process according to any of the preceding claims, wherein rhodium is used as a metal from group 8 or 9 of the periodic table of the elements.

## Revendications

1. Procédé d'hydroformylation d'oléfines en C₂ à C₈ dans au moins un réacteur par utilisation d'un système catalyseur hétérogénéisé, dans lequel
on envoie un mélange d'entrée gazeux, qui contient les oléfines en C₂ à C₈, en même temps qu'un gaz de synthèse dans au moins un réacteur, sur un support disposé dans l'au moins un réacteur, constitué d'un matériau céramique poreux, sur lequel le système catalyseur, qui comprend un métal du 8e ou du 9e groupe du système périodique des éléments, au moins un ligand contenant du phosphore organique, un stabilisant, est présent sous forme hétérogénéisée ; dans lequel
le support est un monolithe, c'est-à-dire un bloc d'un matériau céramique, ou se présente sous forme d'une poudre, sous forme d'un granulé ou sous forme d'objets moulés, et le support est constitué d'un matériau de type carbure, nitrure, siliciure, ou de mélanges de ceux-ci, et dans lequel
l'hydroformylation est mise en œuvre à une température dans le réacteur de 65 à 200 °C ;
**caractérisé en ce que** le procédé comprend les étapes suivantes pour l'arrêt de l'au moins un réacteur :
a) arrêt de l'amenée du mélange d'entrée gazeux pendant qu'en outre le gaz de synthèse est amené dans l'au moins un réacteur ;
b) abaissement de la température dans l'au moins un réacteur à la température ambiante ;
c) arrêt de l'amenée du gaz de synthèse et stockage de l'au moins un réacteur sous une atmosphère d'un gaz de synthèse, jusqu'à redémarrage du procédé d'hydroformylation.

2. Procédé selon la revendication 1, dans lequel, pour arrêter l'amenée du mélange d'entrée gazeux dans l'étape a), on augmente l'amenée de gaz de synthèse.

3. Procédé selon la revendication 1 ou 2, dans lequel on diminue la pression dans l'au moins un réacteur après et/ou pendant l'abaissement de la température dans l'étape b) .

4. Procédé selon la revendication 3, dans lequel l'amenée de gaz de synthèse est déjà diminuée dans l'étape b) .

5. Procédé selon l'une des revendications précédentes, dans lequel, avant l'arrêt de l'amenée du gaz de synthèse dans l'étape c), on augmente de nouveau la pression dans le réacteur.

6. Procédé selon l'une des revendications précédentes, dans lequel le ligand contenant du phosphore organique du système catalyseur d'hydroformylation a de préférence la formule (II)
R'-A-R"-A-R‴ (II)
dans laquelle R', R", R‴ représentent chacun des radicaux organiques, à la condition que le R' et le R‴ ne soient pas identiques, et que les deux A représentent chacun un groupe -O-P(-O)₂- pontant, dans lequel deux des trois atomes d'oxygène -O- sont chacun liés au radical R' et au radical R‴.

7. Procédé selon l'une des revendications précédentes, dans lequel le stabilisant est un composé aminé organique, qui contient au moins un motif 2,2,6,6-tétraméthylpipéridine de formule (I) :

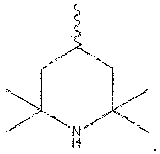

8. Procédé selon l'une des revendications précédentes, dans lequel la céramique de type nitrure est choisie parmi le nitrure de silicium, le nitrure de bore, le nitrure d'aluminium et les mélanges de ceux-ci, la céramique de type carbure est choisie parmi le carbure de silicium, le carbure de bore, le carbure de tungstène ou les mélanges de ceux-ci ; et la céramique de type siliciure est le siliciure de molybdène.

9. Procédé selon la revendication 8, dans lequel le support est constitué d'une céramique de type carbure.

10. Procédé selon la revendication 9, dans lequel le support est constitué de carbure de silicium.

11. Procédé selon l'une des revendications 1 à 7, dans lequel l'hydroformylation est mise en œuvre à une température dans la plage de 75 à 175 °C, de préférence dans la plage de 85 à 150 °C.

12. Procédé selon l'une des revendications précédentes, dans lequel la pression lors de l'hydroformylation est d'au moins 1 bar et n'est pas supérieure à 35 bar, de préférence n'est pas supérieure à 30 bar, plus particulièrement n'est pas supérieure à 25 bar.

13. Procédé selon l'une des revendications précédentes, dans lequel le système catalyseur ne comprend aucun liquide ionique.

14. Procédé selon l'une des revendications précédentes, dans lequel on utilise pour l'hydroformylation dans le procédé des oléfines en C₄.

15. Utilisation selon l'une des revendications précédentes, dans laquelle on utilise en tant que métal du 8e ou du 9e groupe du système périodique des éléments du rhodium.
